# EUROPÄISCHE PATENTANMELDUNG

(11) **EP 3 718 605 A1**
(43) Veröffentlichungstag der Anmeldung: **07.10.2020**
(21) Anmeldenummer: 19167199.9
(22) Anmeldetag: 04.04.2019
(51) Int. Cl.: A62B 18/00

(54) **VORRICHTUNG ZUR ERZEUGUNG EINES LUFTVORHANGS VOR EINEM GESICHT EINES NUTZERS**

(71) Anmelder: Hilti Aktiengesellschaft, 9494 Schaan (LI)
(72) Erfinder: Krämer, Stefan, 86165 Augsburg (DE); Melzer, Lars, 86978 Hohenfurch (DE)
(74) Vertreter: Hilti Aktiengesellschaft Corporate Intellectual Property

(57) **Zusammenfassung**

Die vorliegende Erfindung betrifft eine Vorrichtung (1) zur Erzeugung eines Luftvorhangs (2) vor einem Gesicht (3) eines Nutzers, wobei die Vorrichtung (1) unter anderem eine Pumpe (4) zur Bereitstellung von Luft zur Erzeugung des Luftvorhangs (2) und mindestens eine Düse (5) zur Formung des Luftvorhangs (2) umfasst., wobei die Düse (5) an einer Tragevorrichtung (10) im Mund-Nasen-Bereich des Nutzers angeordnet wird.

## Beschreibung

Die vorliegende Erfindung betrifft eine Vorrichtung zur Erzeugung eines Luftvorhangs vor einem Gesicht eines Nutzers, wobei die Vorrichtung unter anderem eine Pumpe zur Bereitstellung von Luft zur Erzeugung des Luftvorhangs und mindestens eine Düse zur Formung des Luftvorhangs umfasst.

Es sind im Stand der Technik Vorrichtungen bekannt, um Arbeiter, beispielsweise in Kohleminen, oder Bauarbeiter auf Baustellen vor dem Einatmen von verunreinigter Luft zu schützen. In Kohleminen oder auf Baustellen kann die für die Arbeiter zur Verfügung stehende Atemluft insbesondere mit Staub, Schadstoffen oder Schadpartikeln verunreinigt sein. Konventionelle Schutzvorrichtungen, die aus dem Stand der Technik bekannt sind, können zum Beispiel Schutzhelme sein, die auf die Schultern des Arbeiters aufgesetzt werden können, um zu verhindern, dass die Staub- oder Schadpartikel in den Bereich der Nase des Arbeiters gelangen. Diese oft haubenförmig ausgebildeten Schutzvorrichtungen sind zumeist - ähnlich wie ein Taucherhelm - geschlossen. Nachteilig an diesen Vorrichtungen ist, dass sie oft nicht komfortabel zu tragen sind und daher häufig von den Arbeitern nicht akzeptiert werden. Darüber hinaus kann die Beweglichkeit und das Sichtfeld des Arbeiters durch solche geschlossenen Hauben eingeschränkt sein.

Aufgabe der vorliegenden Erfindung ist es, die vorstehend beschriebenen Nachteile konventioneller Schutzvorrichtungen zu überwinden und eine Vorrichtung bereitzustellen, die einen wirksamen Schutz für den Arbeiter gewährleistet, ohne seine Beweglichkeit und sein Sichtfeld einzuschränken. Darüber hinaus soll die bereitzustellende Vorrichtung möglichst leicht und kompakt sein, so dass sie einen möglichst hohen Tragekomfort aufweist und gut von den Arbeitern akzeptiert wird. Ferner soll die bereitzustellende Vorrichtung getragen werden können, unabhängig davon, ob der Nutzer einen Bart oder eine Brille zur Verbesserung der Sehfähigkeit trägt. Es ist ein weiteres Anliegen der Erfindung, den Einatemwiderstand des Nutzers möglichst wenig zu beeinflussen, so dass das Einatmen des Arbeiters durch das Tragen der bereitzustellenden Vorrichtung möglichst nicht erschwert wird.

Die Aufgabe wird gelöst durch den Gegenstand des unabhängigen Anspruchs 1. Vorteilhafte Ausführungsformen zu dem Gegenstand des unabhängigen Anspruchs finden sich in den abhängigen Ansprüchen.

Die Aufgabe wird gelöst durch eine Vorrichtung zur Erzeugung eines Luftvorhangs vor einem Gesicht eines Nutzers. Die Vorrichtung ist dadurch gekennzeichnet, dass die Vorrichtung folgende Bestandteile umfasst:
- mindestens eine Pumpe zur Bereitstellung von Luft zur Erzeugung des Luftvorhangs,
- mindestens eine Düse zur Formung des Luftvorhangs,
wobei die mindestens eine Düse so im Bereich einer Nase des Nutzers an einer Tragevorrichtung angeordnet vorliegt, dass ein Mund-Nase-Bereich des Nutzers von dem Luftvorhang abgedeckt wird. Vorzugsweise wird die Vorrichtung im Sinne der Erfindung auch als Schutzvorrichtung bezeichnet.

Es war vollkommen überraschend, dass eine Schutzvorrichtung bereitgestellt werden kann, bei der der Nutzer der Vorrichtung durch den Luftvorhang atmen kann, wobei die Staub- und/oder Schadpartikel durch den Vorhang abgelenkt werden, so dass der Nutzer möglichst keinen Staub und keine Schadstoffe aus der Atmosphäre einatmet. Dieser Vorteil wird insbesondere durch die geschickte Anordnung der mindestens einen Düse in Bezug auf ein Gesicht des Nutzers erreicht. Insbesondere ermöglicht die vorgeschlagene Anordnung der mindestens einen Düse, dass vorteilhafterweise der gesamte Mund-Nase-Bereich des Nutzers von dem Luftvorhang abgedeckt wird. Mit dem Begriff «Mund-Nase-Bereich des Nutzers» wird im Sinne der Erfindung der Bereich des Gesichts eines Nutzers beschrieben, der sich zwischen Mund und Nase des Nutzers erstreckt und den Mund des Nutzers einschließt. Der Mund-Nase-Bereich ist vorzugsweise im Wesentlichen dreieckig ausgebildet, wobei der Mund eine Unterseite des Mund-Nase-Bereichs festlegt und die Nase die Spitze des im Wesentlichen dreieckig ausgebildeten Mund-Nase-Bereichs. Vorzugsweise ist der Mund-Nase-Bereich größer als die Fläche zwischen Mund und Nase. Mit anderen Worten überragt der Mund-Nase-Bereich die eigentliche Fläche zwischen Mund und Nase und erstreckt sich beispielsweise nach oben bis zur Nasenspitze. Auf der Unterseite des vorzugsweise dreieckig ausgebildeten Mund-Nase-Bereichs überragt der Mund-Nase-Bereich den Mund nach unten und nach rechts und links beispielsweise um jeweils eine Länge von 2 cm.

Vorzugsweise ist auch der Luftvorhang, der von der mindestens einen Düse geformt wird, im Wesentlichen dreieckig ausgebildet, wobei die Form des Luftvorhangs von einem Öffnungswinkel gamma im Bereich der Düse der Vorrichtung gekennzeichnet wird. Dieser Öffnungswinkel kann beispielsweise in einem Bereich von 90 Grad liegen, wobei der Öffnungswinkel zwischen zwei äußeren Luft- oder Randstrahlen des Luftvorhangs gemessen wird. Vorzugsweise wird ein Öffnungswinkel gamma/2 zwischen einer zentral durch das Gesicht des Nutzers verlaufenden virtuellen Achse und einem der beiden äußeren Luftstrahlen des Luftvorhangs festgelegt, wobei der halbe Öffnungswinkel gamma/2 vorzugsweise in einem Bereich von 0 bis 45 Grad liegt.

Der Luftvorhang wird vorzugsweise von einem Luftstrom gebildet, der von der Pumpe der Schutzvorrichtung erzeugt wird. Der Begriff «Luft» bezeichnet im Sinne der Erfindung ein Gasgemisch, das insbesondere Stickstoff N2 und Sauerstoff O2, sowie verschiedene weitere Gase und Gasgemische umfasst. Vorzugsweise wird die Luft von der Pumpe aus der Umgebung des Nutzers eingesaugt und über Leitungsmittel, wie zum Beispiel einen Luftschlauch, an die Düse weitergeleitet. In einer bevorzugten Ausführungsform der Erfindung ist die Tragevorrichtung ein Schutzhelm oder eine Schutzbrille. Wenn die Tragevorrichtung der Schutzvorrichtung als Schutzbrille oder als Schutzhelm ausgebildet ist, ist die Pumpe vorzugsweise auf einer Rückseite der Schutzvorrichtung angeordnet, die vorzugsweise am Hinterkopf des Nutzers angeordnet ist. Vorzugsweise bildet die Tragevorrichtung die Grundstruktur für die Schutzvorrichtung, wobei die verschiedenen Bestandteile der Schutzvorrichtung an der Tragevorrichtung befestigt werden können.

Vorzugsweise ist die Pumpe dazu eingerichtet, Luft im Wesentlichen pulsationsfrei zu fördern und abzugeben, so dass sich vorteilhafterweise eine laminare Strömung ausbildet, die den Luftvorhang bildet. Vorzugsweise ist die Pumpe in Bezug auf den Volumenstrom, der für die Erzeugung des Luftvorhangs erforderlich ist, an die mindestens eine Düse bzw. die Düsenanordnung bzw. ihre Geometrie angepasst. Mit anderen Worten kann die Pumpe im Volumenstrom passend zu der Düse bzw. der Düsenanordnung ausgelegt sein. Es ist im Sinne der Erfindung ganz besonders bevorzugt, dass die Pumpe dazu eingerichtet ist, den Unterdruck, der in einem Filter der Schutzvorrichtung herrscht, zu überwinden. Insbesondere ist es im Sinne der Erfindung bevorzugt, dass die Pumpe durch ihr Funktionsprinzip für einen im Wesentlichen pulsationsfreien Luftstrom sorgt. Beispielsweise können Piezopumpen verwendet werden, die mit Frequenzen in einem Bereich von 30 bis 40 kHz, vorzugsweise mit einer Frequenz von etwa 35 kHz arbeiten. Darüber hinaus können Drehschieberpumpen verwendet werden, die ebenfalls im Wesentlichen pulsationsfrei arbeiten.

Für die Bereitstellung einer laminaren Luftströmung ist es bevorzugt, dass eine Geschwindigkeit innerhalb des Luftstroms nicht zu hoch ausgewählt wird, um eine Entstehung von Wirbeln und turbulenten Strömungen zu verhindern. Es ist im Sinne der Erfindung bevorzugt, dass die Düse auf den Volumenstrom der Pumpe angepasst wird. Im Sinne der Erfindung ist es ferner bevorzugt, dass die Pumpe mit einem Volumenstrom in einem Bereich von 1,0 bis 1,5 l/min betrieben wird, besonders bevorzugt ist ein Volumenstrom von 1,2 l/min. Dieser bevorzugte Volumenstrom kann vorzugsweise mit einer Strömungsgeschwindigkeit in einem Bereich von 1-2 m/s kombiniert werden. Durch diese Kombination von Strömungsgeschwindigkeit und Volumenstrom wird vorteilhafterweise ein laminarer Luftstrom erreicht. Es ist im Sinne der Erfindung insbesondere bevorzugt, dass eine Düsengeometrie dazu eingerichtet ist, dass der Luftstrom möglichst breit aufgefächert wird.

Vorzugsweise dient die Pumpe zur Bereitstellung von Luft zur Erzeugung des Luftvorhangs. Es können zu diesem Zweck insbesondere Drehschieberpumpen oder Piezopumpen verwendet werden. Vor allem Piezopumpen sind besonders kostengünstig und weisen ein geringes Gewicht auf. Es ist im Sinne der Erfindung bevorzugt, dass der Nutzer der Schutzvorrichtung, also beispielsweise der Träger der Schutzbrille oder des Schutzhelms, durch den Luftvorhang atmet. Vorzugsweise werden die feinen Staub- oder Schadpartikel durch den bevorzugt schnell strömenden Luftvorhang abgelenkt. Es ist im Sinne der Erfindung bevorzugt, dass eine Geschwindigkeit des Luftstroms bzw. Luftvorhangs im Bereich vor der Nase des Nutzers der Schutzvorrichtung bei ca. 2 m/s liegt und im Bereich vor dem Mund des Nutzers bei ca. 1 m/s. Vorzugsweise hängt die Strömungsgeschwindigkeit davon ab, mit welcher Geschwindigkeit zumindest ein Teil des Luftstroms bzw. des Luftvorhangs von Mund oder Nase des Nutzers eingesogen wird. Durch die unterschiedlichen Strömungsquerschnitte von Mund und Nase können unterschiedliche Strömungsgeschwindigkeiten im Luftstrom bzw. im Luftvorhang vor dem Mund bzw. vor der Nase des Nutzers erzeugt werden.

Es stellt einen besonderen Vorteil der vorgeschlagenen Schutzvorrichtung dar, dass die Luft im Kontext der vorliegenden Erfindung keinen Widerstand erfährt, so dass der Einatemwiderstand des Nutzers der Schutzvorrichtung nicht erhöht wird. Mit anderen Worten ist es im Sinne der Erfindung bevorzugt, dass der Träger der Schutzbrille oder des Schutzhelms bei Verwendung der Schutzvorrichtung gleich oder zumindest ähnlich gut ein- und ausatmen kann, wie ohne Schutzvorrichtung. Vorteilhafterweise verhindern die Schutzbrille bzw. der Schutzhelm das Eindringen von Staub oder Schadpartikeln von oben.

Vorzugsweise deckt der Luftvorhang einen großen Bereich der unteren Gesichtshälfte des Nutzers ab. In diesem Zusammenhang kann ein Mund-Nase-Bereich des Nutzers definiert werden, der vorzugsweise eine dreieckige Grundform aufweist und ein wenig größer ausgebildet ist als die Fläche, die von Mund und Nase aufgespannt wird. Vorzugsweise gewährleistet die Schutzvorrichtung eine Abdeckung des Mund-Nase-Bereichs mit dem Luftvorhang unabhängig davon, ob äußere, gegebenenfalls störende Luftströmungen oder Bewegungen vorhanden sind. Eine solche optimale Abdeckung kann insbesondere durch das synergistische Zusammenspiel von Pumpe und mindestens einer Düse bzw. Düsenanordnung ermöglicht werden. Es stellt ein besonderes Verdienst der Erfindung dar, dass die Schutzvorrichtung das Einatmen von Staub- und Schadpartikeln besonders wirksam verhindert, unabhängig davon, ob der Träger der Schutzbrille oder des Schutzhelms einen Bart und/oder eine Brille trägt.

Der Luftvorhang wird vorzugsweise von der mindestens einen Düse geformt. Im Sinne der Erfindung ist insbesondere ein Luftstrom bevorzugt, der von einem Öffnungswinkel gamma definiert wird. Die Form des Luftvorhangs kann insbesondere von der Geometrie der mindestens einen Düse bestimmt werden. Es kann im Sinne der Erfindung auch bevorzugt sein, dass die Funktion der Düse von einer Düsenanordnung ausgeführt wird. Die Düsenanordnung kann vorzugsweise zwei oder mehr Düsen umfassen, wobei die einzelnen Düsen insbesondere im Bereich einer Nase des Nutzers der Schutzvorrichtung angeordnet vorliegen. Bevorzugte Düsenanordnungen werden insbesondere in den beigefügten Figuren dargestellt. Um den Luftstrom zu einem Luftvorhang zu formen, können auch andere Bestandteile der Schutzvorrichtung einen Beitrag leisten. Beispielsweise kann die Strömung über Bestandteile einer Schutzbrille geführt werden, wenn die Tragevorrichtung von einer Schutzbrille gebildet wird. Vorzugsweise ist die Düse bzw. die Düsenanordnung dazu eingerichtet, eine laminare Strömung zur Erzeugung des Luftvorhangs zu erzeugen bzw. zu formen. Vorteilhafterweise hat sich gezeigt, dass die Düsen im Kontext der vorliegenden Erfindung besonders verlustfrei arbeiten. Durch die Düse bzw. die Düsenanordnung wird vorteilhafterweise eine im Wesentlichen vollständige Abdeckung der unteren Gesichtshälfte bzw. des Mund-Nase-Bereichs des Nutzers der Schutzvorrichtung ermöglicht. Diese im Wesentlichen vollständige Abdeckung wird insbesondere durch die von der Düse oder der Düsenanordnung bereitgestellte Form des Luftvorhangs ermöglicht, die maßgeblich von der Positionierung der Düsen bzw. der Anordnung der Düsen innerhalb der Schutzvorrichtung beeinflusst wird. In einer ganz bevorzugten Ausführungsform der Erfindung können zwei Düsen an der Schutzvorrichtung vorgesehen sein, wobei die beiden Düsen rechts und links unter einer in einem Helm der Schutzvorrichtung integrierten Schutzbrille angeordnet vorliegen.

Es ist im Sinne der Erfindung bevorzugt, dass die mindestens eine Düse bzw. die Düsenanordnung einen Abstand d von einem Gesicht des Nutzers aufweist, wobei der Abstand d in einem Bereich von 2 bis 30 mm, bevorzugt 5 bis 20 mm und besonders bevorzugt bei 10 mm liegt. Mit anderen Worten ist es im Sinne der Erfindung bevorzugt, dass zwischen Düse bzw. Düsenanordnung und der Nase, insbesondere der Nasenspitze, eines Nutzers ein Abstand besteht. In einer ganz besonders bevorzugten Ausführung der Erfindung beträgt dieser Abstand circa 1 cm. Vorzugsweise bezeichnet der Begriff «Abstand» einen kürzesten Abstand zwischen Nasenspitze des Nutzers der Schutzvorrichtung und der Düse bzw. der Düsenanordnung. Durch den bevorzugten Abstand werden vorteilhafterweise Verwirbelungen minimiert, wie Tests gezeigt haben.

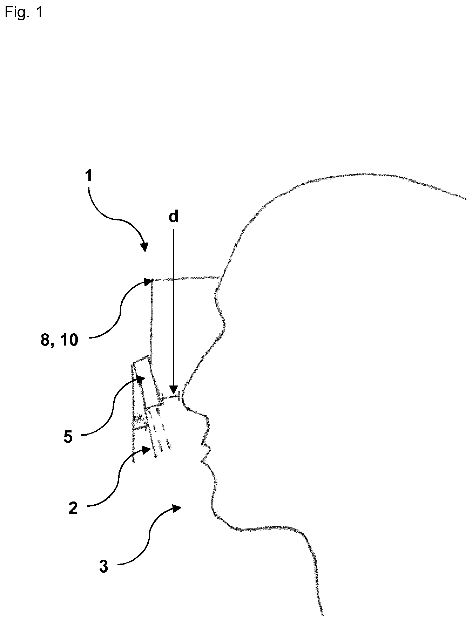

Vorzugsweise ist die mindestens eine Düse bzw. die Düsenanordnung dazu eingerichtet, einen Luftstrom in einem Winkel *alpha* abzugeben, wobei der Winkel *alpha* in einem Bereich von 10 bis 30 Grad liegt. Es ist im Sinne der Erfindung bevorzugt, den Winkel alpha so einzustellen, dass kein Luftspalt am Kinn des Nutzers entsteht. Vorzugsweise wird der Winkel alpha ausgehend von einer vertikalen Achse gemessen. Dabei kann es sich vorzugsweise um die vertikale, vorzugsweise zentral durch das Gesicht des Nutzers verlaufende virtuelle Achse handeln, die das Gesicht des Nutzers gedanklich in zwei Hälften teilt und die beispielsweise bei der Festlegung des Öffnungswinkel gamma/2 verwendet wird. Es ist im Sinne der Erfindung bevorzugt, dass die Düse bzw. die Düsenanordnung in Richtung des Gesichts des Nutzers geneigt ist. Mit anderen Worten kann die Düse bzw. die Düsenanordnung der Schutzvorrichtung auf eine Brust oder einen Bauch des Nutzers gerichtet sein, so dass der erzeugte Luftstrom, der den Luftvorhang bildet, beispielsweise mit einem Abstand von 1 bis 3 cm vor einem Kinn des Nutzers verläuft. Ein bevorzugter Verlauf des Luftstroms, sowie der Abstand d und der Neigungswinkel alpha sind beispielsweise in Figur 1 dargestellt.

Die Einstellung des Winkels *alpha* führt in Kombination mit dem oben genannten Abstand zwischen Düse bzw. Düsenanordnung und Nasenspitze des Nutzers zu einem optimalen Verwirbelungsverhalten innerhalb des Luftstroms. Dies bedeutet im Sinne der Erfindung bevorzugt, dass Verwirbelungen minimiert werden können, wenn ein Abstand d beispielsweise 1 cm beträgt und der Winkel *alpha* zwischen 10 und 30 Grad eingestellt ist. Die genannten Winkel und Abstände können insbesondere in Abhängigkeit von der Kopfform eines Nutzers der Schutzvorrichtung ausgewählt und/oder variiert werden.

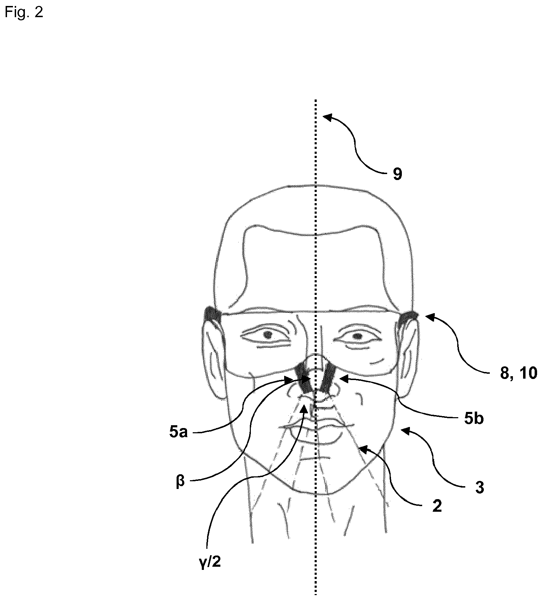

Es ist im Sinne der Erfindung bevorzugt, dass die Vorrichtung zwei Düsen umfasst, die vorzugsweise eine Düsenanordnung bilden. Vorzugsweise schließen die Düsen einen Winkel beta miteinander ein, wobei der Winkel beta in einem Bereich von 0 bis 10 Grad liegt. Insbesondere liegt der Betrag der Winkel in Bezug auf die beiden Düsen in einem Bereich von 0 bis 10 Grad, wobei die Vorzeichen für die Neigungswinkel der beiden Düsen vorzugsweise entgegengesetzt sind. Der Luftvorhang, der vorzugsweise den gesamten Nase-Mund-Bereich des Nutzers abdeckt, wird insbesondere durch die geschickte Anordnung der Düsenanordnung in Bezug auf ein Gesicht des Nutzers erreicht. Diese Anordnung wird insbesondere durch die Winkel *alpha*, *beta* und delta gekennzeichnet. Es ist im Sinne der Erfindung bevorzugt, dass die Düsen in Bezug auf die vertikale, vorzugsweise zentral durch das Gesicht des Nutzers verlaufende virtuelle Achse, die das Gesicht des Nutzers gedanklich in zwei Hälften teilt, geneigt ausgebildet sind. Vorzugsweise sind die Düsen rechts und links von dieser vertikalen Achse angeordnet, wobei die Düsen insbesondere in einer Vorder- bzw. Frontansicht des Gesichts des Nutzers bzw. der Erfindung geneigt ausgebildet sind. Vorzugsweise liegen die beiden Düsen in Bezug auf die virtuelle, vertikale Achse verkippt vor, wobei die Düsen vorzugsweise so verkippt sein können, wie in Figur 2 dargestellt. Insbesondere kann eine erste Düse vor einer linken Gesichtshälfte des Nutzers angeordnet sein, während eine zweite Düse der Düsenanordnung vor einer rechten Gesichtshälfte des Nutzers angeordnet ist. Die Begriffe «rechts» und «links» beziehen sich vorzugsweise auf die virtuelle vertikale Achse entsprechend einer Vorderansicht eines Gesichts eines Nutzers der Schutzvorrichtung. Eine solche Vorderansicht der Erfindung wird beispielsweise in Figur 2 gezeigt. Vorzugsweise können die Begriffe «oben» und «unten» im Kontext der vorliegenden Erfindung so verstanden werden, dass das Kinn einen unteren Bereich des Gesichts des Nutzers bildet, während der obere Teil des Gesichts üblicherweise mit Haaren bedeckt ist. Vorzugsweise weisen die beiden Düsen der Düsenanordnung in dieser Ausführungsform der Erfindung in einem unteren Bereich einen kleineren Abstand zur virtuellen vertikalen Achse auf als im oberen Bereich der Düsen. Mit anderen Worten sind die beiden Düsen wie die Schenkel eines Buchstaben «V» zueinander angeordnet. Der Neigungswinkel beta weist daher für eine der beiden Düsen vorzugsweise ein positives Vorzeichen auf, während der Neigungswinkel beta für die andere Düse der Düsenanordnung ein negatives Vorzeichen aufweist. Vorzugsweise sind die Beträge des Neigungswinkels für die erste Düse und für die zweite Düse gleich oder im Wesentlichen gleich.

Vorzugsweise erzeugt jede der beiden Düsen der Düsenanordnung einen eigenen Luftvorhang. Die beiden separaten Einzel-Luftvorhänge überlagern sich vorzugsweise zu einem Gesamt-Luftvorhang, der vorzugsweise den gesamten Mund-Nase-Raum des Nutzers der Schutzvorrichtung abdeckt. Durch die Vorsehung der beiden Düsen, die jeweils eine Winkel beta mit einer virtuellen vertikalen Achse einschließen, wird eine Überschneidung der Luftvorhänge vorzugsweise in der Mitte des Gesichts des Nutzers erreicht. Somit ist der sich ausbildende Gesamt-Luftvorhang, bzw. der zugrundeliegende Luftstrom, besonders stark im Bereich unterhalb der Nasenlöcher ausgebildet. Dadurch werden die Staub- und Schadpartikel besonders wirksam abgelenkt und aus dem Bereich unterhalb Nase entfernt, so dass sie nicht in die Nase und Atemwege des Nutzers gelangen können. Vorzugsweise spannen die beiden Düsen einen Luftvorhang mit einem Öffnungswinkel von *gamma* kleiner gleich 90 Grad auf. Durch diesen Öffnungswinkel wird vorteilhafterweise eine vollständige Abdeckung des Mundes und des Bereichs unterhalb der Nase eines Nutzers durch den Gesamt-Luftvorhang erreicht.

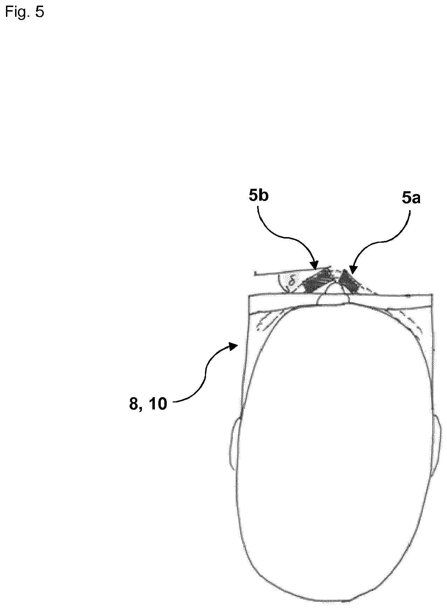

Es ist im Sinne der Erfindung bevorzugt, dass die Düsen mit einer vertikalen Ebene einen Winkel delta einschließen, wobei der Winkel delta in einem Bereich von 10 bis 30 Grad liegt. Die vertikale Ebene kann vorzugsweise die vertikale, vorzugsweise zentral durch das Gesicht des Nutzers verlaufende virtuelle Achse umfassen und im Wesentlichen parallel zu einer Gesichtsebene des Nutzers ausgebildet sein. Die geneigten Düsen können vorzugsweise dem Verlauf der Nasenflügel folgen, wie in Figur 5 dargestellt. Vorzugsweise wird der Winkel delta zwischen den Düsen und der vertikalen Ebene ausgebildet, wobei die Beträge der Neigungswinkel für die erste und die zweite Düse im Wesentlichen gleich sind, während die Vorzeichen der Neigungswinkel für die beiden Düsen vorzugsweise entgegengesetzt sind. Vorzugsweise sind die Düsen auch in einer Top-Ansicht von oben (vgl. Figur 5) v-förmig angeordnet, wobei die Öffnung des Buchstabens «V» in Richtung des Gesichts des Nutzers zeigt, während die Spitze des Buchstaben «V» vom Nutzer wegweist. Mit der Schrägstellung der Düse wird insbesondere eine verbesserte Abdeckung des Gesichts mit dem Luftvorhang erreicht. Dadurch wird vorteilhafterweise vermieden, dass Staubpartikel am Vorhang vorbei vom Nutzer eingeatmet werden können.

Vorzugsweise umfasst die Vorrichtung einen Filter zur Reinigung der Luft, die von der Schutzvorrichtung eingesaugt wird, um den Luftstrom zur Erzeugung des Luftvorhangs zu erzeugen. Es kann bei der Benutzung der Schutzvorrichtung passieren, dass ein Teil des Luftvorhangs vom Nutzer eingeatmet wird. Um sicherzustellen, dass dabei keine Staub- oder Schadpartikel eingeatmet werden, ist es im Sinne der Erfindung bevorzugt, dass die eingesaugte Luft gefiltert wird. Vorzugsweise ist der Filter der Schutzvorrichtung auf einer Rückseite der Vorrichtung angeordnet, so dass der Filter im Bereich des Hinterkopfes des Nutzers angeordnet ist. Mit der Vorsehung des Filters wird vorteilhafterweise auch die Pumpe der Schutzvorrichtung und ihre Mechanik vor Verunreinigungen und Beschädigungen geschützt. Es kann im Sinne der Erfindung bevorzugt sein, Filter der Filterklasse H13 oder HEPA zu verwenden. Vorzugsweise kann die Arbeitszeit mit dem Filter verlängert werden, wenn ein Filter mit einem hohen Abscheidegrad verwendet wird.

Es ist im Sinne der Erfindung bevorzugt, dass die Schutzvorrichtung eine Einheit zur Überwachung einer Funktion der Vorrichtung umfasst. Diese Einheit wird im Sinne der Erfindung bevorzugt auch als Funktionsüberwachungseinheit bezeichnet. Es ist im Sinne der Erfindung bevorzugt, dass die Funktionsüberwachungseinheit elektronisch arbeitet. Vorzugsweise kann die Funktionsüberwachungseinheit Bestandteil einer Steuereinheit der vorgeschlagenen Schutzvorrichtung sein. Vorzugsweise erfüllt die Funktionsüberwachungseinheit die Anforderungen an eine Warnvorrichtung, die zum Teil gesetzlich gefordert wird, um festzustellen, dass ein Atemschutzgerät bzw. die Schutzvorrichtung ausfällt bzw. fehlerhaft arbeitet. Vorzugsweise kann die Funktionsüberwachungseinheit eine Volumenstromüberwachung durchführen, wobei eine solche Volumenstromüberwachung in Verbindung mit dem Luftvorhang schwierig sein kann. Es ist daher im Sinne der Erfindung ganz besonders bevorzugt, dass eine Überwachung eines Differenzdrucks zwischen Filtergehäuse und Umwelt bzw. Umgebung des Nutzers durchgeführt wird. Mit anderen Worten ist die Funktionsüberwachungseinheit dazu eingerichtet, einen Differenzdruck zwischen Schutzvorrichtung und Umgebung des Nutzers zu überwachen. Vorzugsweise kann die Funktionsüberwachungseinheit darüber hinaus dafür verwendet werden, einen Filterzustand des Filters der Schutzvorrichtung zu überwachen.

Im Kontext der vorgeschlagenen Funktionsüberwachung ist es im Sinne der Erfindung bevorzugt, dass die Funktionsüberwachungseinheit in der Lage ist, den verwendeten Filter zu charakterisieren bzw. zu erkennen. Ferner ist es vorgesehen, dass die Funktionsüberwachungseinheit einen Druckabfall durch den Filter in einem unverbrauchten und gegebenenfalls einem verstopften bzw. zugesetzten Zustand ermitteln kann. Insbesondere verfügt die Funktionsüberwachungseinheit über Mittel, mit denen die ermittelten Werte miteinander verglichen werden können. In dem Fall, dass eine Druckdifferenz gemessen wird, die beispielsweise zwischen dem Wert "null" und dem für einen neuen, unverbrauchten Filter gemessenen Druckdifferenzwert liegt, kann es sein, dass ein Fehler an der Pumpe und/oder an der Stromversorgung vorliegt. Ein solches Messergebnis kann auch dahingehend verstanden werden, dass ein Leck in der Schutzvorrichtung bzw. ihren Leitungsmitteln vorliegt. Wenn eine ermittelte Druckdifferenz zwischen einem ersten Wert, der für einen neuen, unverbrauchten Filter gemessen wurde, und einem zweiten Wert liegt, der für einen verstopften Zustand gemessen wurde, kann von einem im Wesentlichen fehlerfreien Funktionieren der Schutzvorrichtung ausgegangen werden. Wenn der Wert für den Differenzdruck über einem bestimmten Grenzwert liegt, kann es sein, dass der Filter verstopft ist und ersetzt werden sollte. Um im Kontext der vorgeschlagenen Funktionsüberwachung den Differenzdruck zu messen, können insbesondere Differenzdrucksensoren verwendet werden.

Vorzugsweise umfasst die Schutzvorrichtung eine Energieversorgungseinrichtung zur Bereitstellung von Energie für die Bestandteile der Vorrichtung. Dabei kann es sich vorzugsweise um eine Strom- und/oder Spannungsquelle handeln. Die Energieversorgungseinrichtung ist vorzugsweise dazu eingerichtet, die Schutzvorrichtung mit ausreichend elektrischer Energie zum Betrieb der Bestandteile zu versorgen, wie beispielsweise die Pumpe, die Düsen, den Filter und/oder die Funktionsüberwachungseinheit. Die Energieversorgungseinrichtung kann beispielsweise von einer Batterie oder einem oder mehreren Akkumulatoren gebildet werden. Beispielsweise können im Kontext der vorliegenden Erfindung Akkus mit einer Spannung von 12 Volt verwendet werden.

Es hat sich gezeigt, dass für die Funktion der Schutzvorrichtung, d.h. insbesondere die Ablenkungswirkung des Luftvorhangs in Bezug auf Staub- oder Schadpartikel, insbesondere die Düse bzw. die Düsenanordnung relevant ist. Der Filter der Schutzvorrichtung kann vorzugsweise am Hinterkopf des Nutzers platziert sein. Dies ist mit dem Vorteil verbunden, dass die Luft am Hinterkopf des Nutzers häufig sauberer ist als die Luft vor dem Gesicht des Nutzers, wobei dieser Bereich oft mit dem Arbeitsbereich des Nutzers zusammenfällt. Die Funktionsüberwachungseinheit arbeitet vorzugsweise basierend auf einer Differenzdruckmessung. Sie kann beispielsweise in einem Filterkasten innerhalb der Schutzvorrichtung angeordnet sein. Es ist im Sinne bevorzugt, dass die Energieversorgungseinrichtung möglichst tief innerhalb der Schutzvorrichtung angeordnet vorliegt. Dadurch wird eine optimale Verteilung des Gewichts der Energieversorgungseinrichtung erreicht, insbesondere, wenn die Energieversorgungseinrichtung von Akkumulatoren gebildet wird. Vorzugsweise kann die Schutzvorrichtung auch eine Stromversorgungseinrichtung umfassen, die beispielsweise mit der Pumpe der Schutzvorrichtung gekoppelt sein kann. Die Stromversorgungseinrichtung und/oder die Pumpe der Schutzvorrichtung sind vorzugsweise ebenfalls möglichst tief innerhalb der Vorrichtung angeordnet. Es kann in einer Ausführungsform der Erfindung bevorzugt sein, dass die Stromversorgungseinrichtung und/oder die Pumpe zum Zwecke des Gewichtsausgleichs gegenüber den Akkumulatoren angeordnet sind.

Es ist im Sinne der Erfindung insbesondere bevorzugt, dass die Schutzvorrichtung über Mittel verfügt, um die von den Akkumulatoren bevorzugt abgegebene Gleichspannung in eine Wechselspannung umzuwandeln, die für den Betrieb von Piezopumpen benötigt wird. Vorzugsweise ist die Stromversorgungseinrichtung der Schutzvorrichtung dazu eingerichtet, die vom Akkumulator abgegeben Spannung so zu verändern, dass sie für den Betrieb der Piezopumpen verwendet werden kann, wenn als Pumpen Piezopumpen verwendet werden.

Weitere Vorteile ergeben sich aus der folgenden Figurenbeschreibung. Die Figuren, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

In den Figuren sind gleiche und gleichartige Komponenten mit gleichen Bezugszeichen beziffert. Es zeigen:
- Fig. 1: Seitenansicht einer bevorzugten Ausgestaltung der Erfindung
- Fig. 2: Vorderansicht einer bevorzugten Ausgestaltung der Erfindung
- Fig. 3: schematische Seitenansicht einer bevorzugten Ausgestaltung der Erfindung
- Fig. 4: schematische Seitenansicht einer bevorzugten Ausgestaltung der Erfindung
- Fig. 5: Draufsicht einer bevorzugten Ausgestaltung der Erfindung

### Ausführungsbeispiele:

Figur 1 zeigt eine bevorzugte Ausgestaltung einer vorgeschlagenen Schutzvorrichtung (1). Dargestellt ist eine Tragevorrichtung (10), die insbesondere als Schutzbrille (8) ausgebildet sein kann. Die Tragevorrichtung (10) trägt eine Düse (5) oder eine Düsenanordnung (5), wobei die Düse (5) oder die Düsenanordnung (5) dazu eingerichtet ist, einen Luftvorhang (2) vor dem Gesicht (3) eines Nutzers der Schutzvorrichtung (1) zu formen. Die Düse (5) oder die Düsenanordnung (5) ist vorzugsweise in dem Sinne geneigt ausgebildet, dass sie in einem unteren Bereich einen geringeren Abstand d zum Gesicht (3) des Nutzers aufweist als in einem oberen Bereich.

Mit anderen Worten kann die Düse (5) oder die Düsenanordnung (5) mit einem unteren Bereich in Richtung des Gesichts (3) des Nutzers verkippt vorliegen. Diese Verkippung oder Neigung der Düse (5) oder der Düsenanordnung (5) wird durch den Neigungswinkel alpha beschrieben, der vorzugsweise in einem Bereich von 10 bis 30 Grad liegt.

Figur 2 zeigt eine Vorderansicht einer bevorzugten Ausgestaltung der vorgeschlagenen Schutzvorrichtung (1). Die in Figur 2 dargestellte bevorzugte Ausführungsform der Schutzvorrichtung (1) ist als Schutzbrille (8) ausgebildet, wobei die Düsenanordnung (5) vorzugsweise unterhalb der Brillengläser, bzw. unterhalb eines Übergangsbereich zwischen den Brillenglasabschnitten, angeordnet vorliegt. Durch diesen Mittelbereich der Schutzbrille (8) verläuft vorzugsweise auch eine vertikale gedankliche Achse (9), die das Gesicht (3) des Nutzers in zwei Gesichtshälften teilt.

Insbesondere zeigt Figur 2 eine Düsenanordnung (5), die zwei Einzeldüsen (5a, 5b) umfasst. Die erste Einzeldüse (5a) kann beispielsweise auf der linken Gesichtshälfte in Bezug auf eine vertikale virtuelle Achse (9) angeordnet sein, während eine zweite Einzeldüse (5b) auf der rechten Gesichtshälfte des Nutzers in Bezug auf die vertikale virtuelle Achse (9) angeordnet liegt. Die beiden Einzeldüsen (5a, 5b) sind in Bezug auf die vertikale virtuelle Achse (9) geneigt ausgebildet, wobei die beiden Einzeldüsen (5a, 5b) vorzugsweise achsensymmetrisch angeordnet sind. Die vertikale virtuelle Achse (9) kann dabei vorzugsweise als Symmetrieachse fungieren. Die Neigung der beiden Einzeldüsen (5a, 5b) äußert sich beispielsweise darin, dass die Einzeldüsen (5a, 5b) in einem unteren Bereich einen kleinen Abstand zu der vertikalen virtuellen Achse (9) aufweisen als in einem oberen Bereich. Die Neigung der beiden Einzeldüsen (5a, 5b) wird vorzugsweise durch den Neigungswinkel beta beschrieben, wobei der Winkel beta in einem Bereich von 0 bis 10 Grad liegt.

Die Einzeldüsen (5a, 5b) geben jeweils einen Luftvorhang (2) ab, wobei die beiden Luftvorhänge (2) zusammen den Gesamt-Luftvorhang (2) bilden. Die äußeren Randstrahlen des Luftvorhangs (2) gehen von den Ausgängen der Düsen (5) ab und spannen den bevorzugt dreieckig ausgebildeten Luftvorhang (2) auf. Die äußeren Randstrahlen des Luftvorhangs (2) verlaufen vorzugsweise symmetrisch zueinander, wobei die vertikale virtuelle Achse (9) vorzugsweise als Symmetrieachse fungiert. Die Größe des durch die äußeren Randstrahlen aufgespannten Luftvorhangs (2) wird durch den Öffnungswinkel gamma festgelegt, wobei der Winkel gamma dem gesamten Öffnungswinkel, der von den beiden äußeren Randstrahlen des Luftvorhangs (2) eingeschlossen wird, entspricht. Die vertikale virtuelle Achse (9) teilt den Öffnungswinkel gamma vorzugsweise in zwei gleich große Hälften auf, die jeweils den Betrag «gamma/2» aufweisen. Mit anderen Worten erstreckt sich der «halbe Öffnungswinkel gamma/2» zwischen der vertikalen virtuellen Achse (9) und je einem der äußeren Randstrahlen des Luftvorhangs (2).

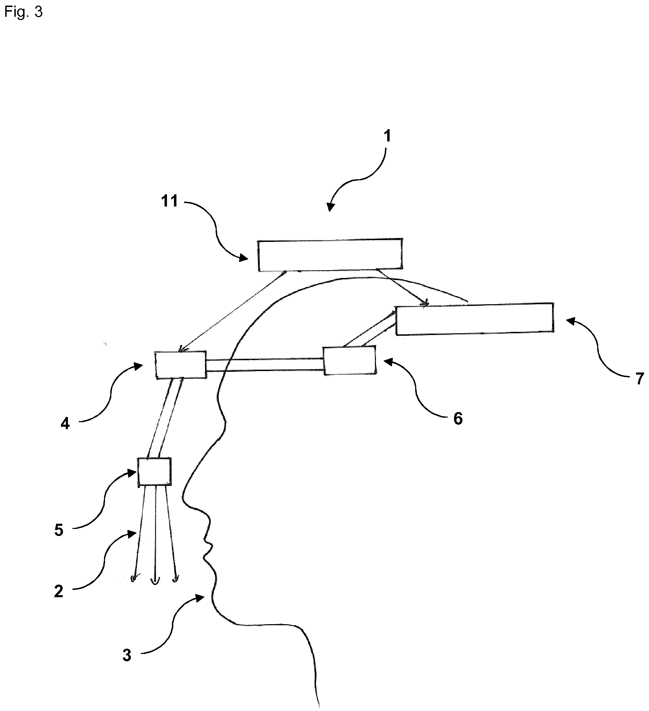

Figur 3 zeigt eine schematische Seitenansicht einer bevorzugten Ausgestaltung der Schutzvorrichtung (1). Die in Figur 3 dargestellte bevorzugte Ausführungsform der Schutzvorrichtung (1) kann als Schutzhelm ausgebildet sein, wobei der Schutzhelm dazu eingerichtet ist, während der Arbeit von einem Arbeiter getragen zu werden. Dargestellt ist eine Düse (5) oder eine Düsenanordnung (5), die dazu eingerichtet ist, einen Luftvorhang (2) vor dem Gesicht (3) eines Trägers der Schutzvorrichtung (1) zu erzeugen. Der Luftstrom, mit dem der Luftvorhang (2) erzeugt wird, kommt dabei vorzugsweise von einer Pumpe (4), die in der in Figur 3 dargestellten bevorzugten Ausführungsform der Erfindung an dem Helm der Schutzvorrichtung (1) befestigt sein kann. Die Pumpe (4) kann in anderen Ausführungsformen der Erfindung auch an anderen Orten innerhalb der Vorrichtung (1) angeordnet sein. Vorzugsweise ist die Pumpe (4) über Leitungsmittel mit der Düse (5) bzw. der Düsenanordnung (5) verbunden, so dass der Luftstrom, der von der Pumpe (4) erzeugt wird, an die Düse (5) oder die Düsenanordnung (5) weitergeleitet werden kann. Die Leistungsmittel können vorzugsweise als Luftschläuche ausgebildet sein. Die Pumpe (4) kann darüber hinaus mit Leitungsmitteln mit einem Filter (6) verbunden sein. Mit dem Filter (6) kann die von der Pumpe (4) eingesaugte Luft gereinigt werden, bevor sie als Luftvorhang (2) in den Mund-Nase-Bereich des Nutzers geblasen wird. Vorzugsweise verlängert die durch den Filter gereinigte Luft auch die Laufzeit der vorgeschlagenen Schutzvorrichtung (1), da durch die Filterung der Luft die Mechanik der Pumpe (4) geschont wird. Die Pumpe (4) wird mit einer Energieversorgungseinrichtung (11) mit elektrischer Energie versorgt. Dabei handelt es sich vorzugsweise um einen wiederaufladbaren Akkumulator, so dass auf die Vorsehung eines kabelgeführten Netzanschlusses zur Stromversorgung verzichtet werden kann. Die Energieversorgungseinrichtung (11) und der Filter (6) können vorzugsweise mit einer Funktionsüberwachungseinheit (7) verbunden vorliegen, wobei die Funktionsüberwachungseinheit (7) vorzugsweise dazu eingerichtet ist, die Bestandteile der Vorrichtung (1) hinsichtlich ihrer Funktionalität zu überwachen und gegebenenfalls zu steuern.

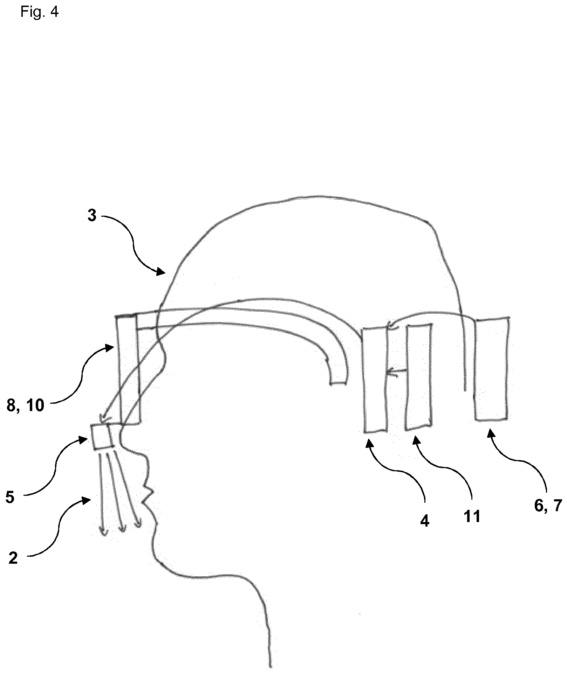

Figur 4 zeigte eine schematische Seitenansicht einer weiteren bevorzugten Ausgestaltung der Schutzvorrichtung (1). Das in Figur 4 dargestellte Ausführungsbeispiel stellt eine Schutzbrille (8) dar, die der Nutzer vor den Augen trägt. Unterhalb der Brillengläser ist die Düse (5) oder die Düsenanordnung (5) angeordnet, mit der der Luftstrom zur Erzeugung des Luftvorhangs (2) in den Mund-Nase-Bereich des Nutzers geblasen werden kann. Auf einer Rückseite des Kopfes des Nutzers können weitere Bestandteile der Schutzvorrichtung (1) angeordnet sein. Wenn die Schutzvorrichtung (1) - wie in Figur 4 - als Schutzbrille (8) ausgebildet ist, kann die Vorrichtung (1) einen Gurt, der beispielsweise von einem elastischen Band gebildet wird, umfassen, der um den (Hinter-)Kopf des Nutzers gelegt oder geführt wird und dort die weiteren Bestandteile der Schutzvorrichtung (1) umfasst, während der vordere Teil der Schutzvorrichtung (1) von der Schutzbrille (8) gebildet wird. Bei den weiteren Bestandteilen der Schutzvorrichtung (1), die beispielsweise im Bereich des Hinterkopfes des Nutzers angeordnet sein können, kann es sich beispielsweise um die Pumpe (4), die Energieversorgungseinrichtung (11), den Filter (6) und/oder die Funktionsüberwachungseinheit (7) handeln.

Figur 5 zeigt die Draufsicht einer bevorzugten Ausgestaltung der Schutzvorrichtung (1), also eine Darstellung der Erfindung von oben. Das in Figur 5 dargestellte Ausführungsbeispiel der Schutzvorrichtung (1) ist als Schutzbrille (8) ausgebildet. Durch die Gläser der Schutzbrille (8) kann eine vertikale Ebene (nicht eingezeichnet) festgelegt werden, die gedanklich im Wesentlichen parallel zu einer Gesichtsfläche des Nutzers ausgebildet ist. Wie in Figur 5 dargestellt, können die beiden Düsen (5a, 5b) der Düsenanordnung (5) des dargestellten Ausführungsbeispiels der Erfindung zu dieser vertikalen Ebene geneigt ausgebildet sein und beispielsweise so angeordnet sein wie die Schenkel eines Buchstaben «V». Die Spitze des Buchstaben «V», der von den beiden Einzeldüsen (5a, 5b) gebildet wird, weist vorzugsweise vom Nutzer fort, während die Öffnung des Buchstaben «V» in Richtung des Gesichts (3) des Nutzers weist. Vorzugsweise folgen die Düsen (5a, 5b) dem Verlauf der Nasenflügel des Nutzers. Die Schrägstellung der Düsen (5a, 5b) der Düsenanordnung (5) kann durch den Winkel delta beschrieben werden, der von der vertikalen virtuellen Ebene und den Düsen (5a, 5b) eingeschlossen wird. Vorzugsweise entsprechen sich die Winkel für die beiden Düsen (5a, 5b) hinsichtlich ihres Betrags und sind in Bezug auf ihr Vorzeichen entgegengesetzt. Mit anderen Worten weist die eine Düse (zum Beispiel 5a) einen positiven Neigungswinkel delta auf, während die anderen Düse (zum Beispiel 5b) einen negativen Neigungswinkel delta aufweist, oder umgekehrt.

### Bezugszeichenliste

- 1: Vorrichtung
- 2: Luftvorhang
- 3: Gesicht
- 4: Pumpe
- 5: Düse
- 6: Filter
- 7: Funktionsüberwachungseinheit
- 8: Schutzbrille
- 9: vertikale virtuelle Achse
- 10: Tragevorrichtung
- 11: Energieversorgungseinrichtung
- d: Abstand zwischen Düse und Nasenspitze

## Patentansprüche

1. Vorrichtung (1) zur Erzeugung eines Luftvorhangs (2) vor einem Gesicht (3) eines Nutzers,
**dadurch gekennzeichnet, dass**
die Vorrichtung (1) folgende Bestandteile umfasst:
- mindestens eine Pumpe (4) zur Bereitstellung von Luft zur Erzeugung des Luftvorhangs (2),
- mindestens eine Düse (5) zur Formung des Luftvorhangs (2),
wobei die mindestens eine Düse (5) so im Bereich einer Nase des Nutzers an einer Tragevorrichtung (10) angeordnet vorliegt, dass ein Mund-Nase-Bereich des Nutzers von dem Luftvorhang (2) abgedeckt wird.

2. Vorrichtung (1) nach Anspruch 1
**dadurch gekennzeichnet, dass**
die Vorrichtung (1) einen Filter (6) umfasst.

3. Vorrichtung (1) nach Anspruch 1 oder 2
**dadurch gekennzeichnet, dass**
die Tragevorrichtung (10) ein Schutzhelm oder eine Schutzbrille (8) ist.

4. Vorrichtung (1) nach einem der vorhergehenden Ansprüche
**dadurch gekennzeichnet, dass**
die Vorrichtung (1) eine Einheit (7) zur Überwachung einer Funktion der Vorrichtung (1) umfasst.

5. Vorrichtung (1) nach einem der vorhergehenden Ansprüche
**dadurch gekennzeichnet, dass**
die mindestens eine Düse (5) einen Abstand d von einem Gesicht (3) des Nutzers aufweist, wobei der Abstand d in einem Bereich von 2 bis 30 mm, bevorzugt 5 bis 20 mm und besonders bevorzugt bei 10 mm liegt.

6. Vorrichtung (1) nach einem der vorhergehenden Ansprüche
**dadurch gekennzeichnet, dass**
die mindestens eine Düse (5) dazu eingerichtet ist, einen Luftstrom in einem Winkel *alpha* abzugeben, wobei der Winkel *alpha* in einem Bereich von 10 bis 30 Grad liegt.

7. Vorrichtung (1) nach einem der vorhergehenden Ansprüche
**dadurch gekennzeichnet, dass**
die Vorrichtung (1) zwei Düsen (5a, 5b) umfasst, wobei die Düsen (5a, 5b) einen Winkel beta miteinander einschließen und wobei der Winkel beta in einem Bereich von 0 bis 10 Grad liegt.

8. Vorrichtung (1) nach einem der vorhergehenden Ansprüche
**dadurch gekennzeichnet, dass**
die Vorrichtung (1) zwei Düsen (5a, 5b) umfasst, wobei die Düsen (5a, 5b) mit einer vertikalen Ebene einen Winkel delta einschließen, wobei der Winkel delta in einem Bereich von 10 bis 30 Grad liegt.

9. Vorrichtung (1) nach einem der vorhergehenden Ansprüche
**dadurch gekennzeichnet, dass**
die Vorrichtung (1) eine Energieversorgungseinrichtung (11) zur Bereitstellung von Energie für die Bestandteile (4, 5, 6, 7) der Vorrichtung (1) umfasst.
